# EUROPEAN PATENT APPLICATION

(11) **EP 4 825 979 A1**
(43) Date of publication of application: **21.10.2026**
(21) Application number: 25890383.0
(22) Date of filing: 28.03.2025
(51) Int. Cl.: C12Q 1/6806, C12Q 1/6869, C40B 40/02, C40B 50/06

(54) **METHOD FOR CONSTRUCTING MICROBIAL SINGLE-CELL SEQUENCING LIBRARY, OBTAINED SEQUENCING LIBRARY AND USE THEREOF**

(30) Priority: 28.02.2025 CN 202510234983
(71) Applicant: ShanghaiTech University, Shanghai 201210 (CN)
(72) Inventor: LIU, Yifan, Pudong New Area, Shanghai 201210 (CN); LIU, Jie, Pudong New Area, Shanghai 201210 (CN); DU, Yanan, Pudong New Area, Shanghai 201210 (CN)
(74) Representative: Canzler & Bergmeier Patentanwälte Partnerschaft mbB
(86) International application number: PCT/CN2025/086041
(87) International publication number: WO 2026/178950

(57) **Abstract**

The present disclosure provides a method for constructing a microbial single-cell sequencing library, a sequencing library, and a use thereof. In the present disclosure, microbial single-cell capture, lysis, whole-genome amplification, fragmentation, and DNA indexing are achieved in microchambers, and the microchambers are then dissolved so that the DNA is present in an aqueous system for purification and enrichment amplification. The present disclosure optimizes the formulation of lysis buffers to achieve thorough microbial lysis without lysis bias. In DNA indexing, introduction of hydrogel beads carrying barcode sequences can significantly improve the pairing efficiency between hydrogel droplets and barcode beads. In addition, it has been found that microbial single-cell indexing achieved by linear amplification is significantly superior to that achieved by exponential amplification. Libraries constructed by the method of the present disclosure are then sequenced, and the results show that, for more than 95% of microbial single cells, the single-cell purity is higher than 95%, and the single-cell genome coverage can reach higher than 80%.

## Description

### Background of the Present Disclosure

### FIELD OF THE DISCLOSURE

The present disclosure relates to the technical field of microbial single-cell sequencing, and in particular, to a method for constructing a microbial single-cell sequencing library, a sequencing library, and a use thereof.

### DESCRIPTION OF RELATED ARTS

Microorganisms are the most abundant and diverse forms of life on Earth. Current studies indicate that there may be up to one trillion microbial species. They are widely distributed in natural environments and in the human body. However, microorganisms in most ecosystems remain insufficiently studied, constituting a substantial portion of so-called "microbial dark matter". Even within the human body, although progress has been made through the Human Microbiome Project, our understanding of microbial communities remains limited. To understand these complex biological communities, it is necessary to elucidate the biological identity and function of each microbial strain, or even of individual cells.

A key determinant of the identity and function of each microbial cell is its genomic information. In existing studies, microbial genomic information is typically obtained by culturing and sequencing isolated strains or by metagenomic sequencing. However, these approaches are inefficient for resolving complex communities: the former is constrained by culturability and resource requirements, whereas the latter lacks strain-level and/or genome-level resolution. With recent advances in high-throughput biotechnology, it has become possible to perform genomic sequencing of individual microbial cells, typically by using microfluidics to isolate cells and then separately amplifying and indexing each genome. Nevertheless, existing methods recover only a small fraction of individual genomes and are not readily scalable due to heavy reliance on continuous microfluidic analysis. Accordingly, there remains a need for methods that provide high coverage and are easy to implement for characterizing complex microbial communities in diverse environments.

### SUMMARY OF THE PRESENT DISCLOSURE

In view of the above-mentioned drawbacks of the prior art, the present disclosure aims to provide a method for constructing a microbial single-cell whole-genome sequencing library, thereby addressing the problems existing in the prior art.

To achieve the above objective and other related objectives, the present disclosure is implemented by the following technical solutions.

In a first aspect, the present disclosure provides a method for constructing a microbial single-cell whole-genome sequencing library, including the following:
1) providing microchambers each containing a single microbial cell, lysing the single cell, and amplifying a whole genome of the microbial single cell;
2) fragmenting the whole genome obtained in step 1), thereby obtaining microchambers each containing a fragmented genome;
3) introducing a barcode bead into each of the microchambers obtained in step 2), and barcoding the fragmented genome, thereby obtaining microchambers each containing a barcoded whole genome; and
4) dissolving the microchambers obtained in step 3), and performing enrichment amplification of the barcoded whole genome, thereby obtaining the microbial single-cell whole-genome sequencing library.

In some embodiments, the microchambers may be gel beads or gel droplets. The gel droplets are microdroplets containing a gel material (e.g., a hydrogel) generated using droplet microfluidic technology. The microdroplets are completely enclosed microchambers, and upon demulsification, the microdroplets are converted into gel beads. The gel beads are permeable microchambers.

In some embodiments, the method for lysing the single cell includes an enzymatic lysis method, an alkaline lysis method, and a heat-shock method.

In some embodiments, enzymes used in the enzymatic lysis method may include one or more of Labiase lysing enzyme, lysostaphin, lysozyme, mutanolysin, zymolyase, and proteinase K.

In some embodiments, reagents for lysing the single cell include a lysis buffer A and a lysis buffer B.

In some embodiments, steps of lysing the single cell include mixing the lysis buffer A with the microchambers to perform a first lysis, washing, and then adding the lysis buffer B to perform a second lysis.

In some embodiments, conditions of the first lysis are 30-43°C, 1300-1800 rpm, and/or 8-16 h.

In some embodiments, conditions of the second lysis are 50-65°C, 1300-1800 rpm, and/or 0.5-2 h.

In some embodiments, amplification of the whole genome of the single cell in step 1) and the enrichment amplification in step 4) include, but are not limited to, exponential amplification or linear amplification, such as PCR, RCA, MALBAC, MDA, or LIANTI.

In some embodiments, genome fragmentation may be performed using an enzymatic fragmentation method or a non-enzymatic fragmentation method.

In some embodiments, the barcode bead includes at least one of a polyacrylamide bead, a polystyrene bead, a poly(ethylene glycol) diacrylate bead, a poly(lactic-co-glycolic acid) bead, an agarose bead, and a magnetic bead.

In some embodiments, in step 3), barcoding of the fragmented genome includes barcoding the fragmented genome by DNA ligation using a DNA ligase, or by single-primer amplification.

In some embodiments, the single-primer amplification is linear amplification. Conventional PCR typically adopts exponential amplification. However, due to the particular structure of the permeable microchambers, exponential amplification is not suitable within the permeable microchambers. Specifically, after whole-genome amplification, the confined space of each microchamber is already filled with nucleic acid molecules, and thus it is unnecessary to increase the amount of nucleic acid molecules by exponential amplification. On the contrary, if both forward and reverse primers are present within a restricted space, exponential amplification is more likely to generate primer dimers, thereby generating non-specific amplification products. Accordingly, the present disclosure innovatively adopts linear amplification to achieve indexing of microbial single cells. Linear amplification requires only a single primer at one end and can also avoid non-specific amplification.

In some embodiments, before barcoding the fragmented genome, the method further includes releasing barcode sequences from the barcode beads.

In some embodiments, the method of releasing the barcode sequences from the barcode beads includes chemical bond cleavage, enzymatic degradation, or ultraviolet (UV) cleavage.

In some embodiments, the chemical bond includes a disulfide bond.

In some embodiments, reagents for the chemical bond cleavage include one or more of dithiothreitol (DTT), dichloromethane, ethyl acetate, acetone, phosphate-buffered saline (PBS), and a degradation enzyme.

In some embodiments, before performing enrichment amplification, the method further includes purifying the barcoded whole genome.

In a second aspect, the present disclosure further provides a microbial single-cell whole-genome sequencing library, which is obtained by using the construction method described above.

In a third aspect, the present disclosure further provides a use of the method for constructing the sequencing library described above or the single-cell whole-genome sequencing library described above in microbial single-cell sequencing.

In a fourth aspect, the present disclosure further provides a method for microbial single-cell whole-genome sequencing, wherein the method includes:
a) constructing a sequencing library according to the method for constructing the microbial single-cell whole-genome sequencing library described above; and
b) sequencing the sequencing library provided in step a).

In some embodiments, the sequencing method may be next-generation sequencing or third-generation sequencing.

In a fifth aspect, the present disclosure further provides a product for constructing a microbial single-cell whole-genome sequencing library, wherein the product includes the aforementioned barcode beads, a reagent for lysing the single cell, a reagent for amplifying the whole genome of the microbial single cell, a fragmentation reagent, a reagent for barcoding the fragmented genome, and a reagent for enrichment amplification.

In some embodiments, the product may be a kit, a chip, or a detection system.

In a sixth aspect, the present disclosure provides a use of the method for constructing the single-cell whole-genome sequencing library described above, or the sequencing library described above, or the method for microbial single-cell whole-genome sequencing described above, or the product described above, in the preparation of a detection kit, a detection device, or a detection system for microbial research.

### Beneficial effects:

The present disclosure provides a method for constructing a microbial single-cell whole-genome sequencing library. The method performs single-cell capture, lysis, whole-genome amplification, fragmentation, and DNA indexing within microchambers. The microchambers are then dissolved, followed by genome purification and enrichment amplification. The present disclosure optimizes the formulation of lysis buffers to achieve thorough microbial lysis without lysis bias. During DNA indexing, introducing hydrogel beads containing barcode sequences can significantly improve the pairing efficiency between the hydrogel microchambers and the barcode beads. In addition, it has been surprisingly found by the present disclosure that single-cell indexing achieved by linear amplification provides a markedly better effect than exponential amplification.

Further, in the method for constructing the microbial single-cell whole-genome sequencing library of the present disclosure, preparation of microchambers, whole-genome amplification, preparation of barcode beads, and DNA indexing are carried out in gel droplets, while the remaining steps can be completed by placing gel beads in an aqueous solution system. In particular, the gel droplets used for whole-genome amplification may also be formed by pipetting, vortexing, or the like. This approach can significantly reduce operational difficulty and time, and can also reduce steps requiring microfluidics and avoid cross-contamination in an open environment.

At present, microbial single-cell whole-genome sequencing technologies mainly include SiC-seq and Microbe-seq, with SiC-seq achieving about 1% single-cell genome coverage and Microbe-seq achieving about 5%-10% single-cell genome coverage. Libraries constructed by the method of the present disclosure and then sequenced, and the results show that, for more than 95% of single cells, the single-cell purity is higher than 95%, and the single-cell genome coverage can reach higher than 80%.

The method for constructing the microbial single-cell whole-genome sequencing library provided by the present disclosure overcomes limitations of droplet-based single-cell genomics and provides a simplified and scalable method for studying microbial communities, while improving genome coverage.

### BRIEF DESCRIPTION OF THE DRAWINGS

Fig. 1 is a schematic diagram of the workflow for constructing and sequencing a microbial single-cell whole-genome sequencing library according to the present disclosure.
Fig. 2 shows microscopic images of microdroplets and hydrogel microchambers in Example 1 of the present disclosure.
Fig. 3 shows staining results after microbial single-cell encapsulation (a), lysis (b), and whole-genome amplification (c) in Example 1.
Fig. 4 shows verification results of sequence integrity of hydrogel barcode beads, in which (a) shows fluorescence (probe) microscopy results and (b) shows statistics of fluorescence intensity.
Fig. 5 shows a microfluidic chip used for single-cell indexing in Example 1, in which (a) shows the microfluidic chip, ①-④ are microscopic images of droplet generation, and (b) shows an image and a statistical histogram of co-encapsulated droplets.
Fig. 6 shows a microscopic image of co-encapsulation of hydrogel barcode beads and hydrogel microchambers.
Fig. 7 shows a fragment size distribution of a single-cell genomic sequencing library in Example 1.
Fig. 8 shows analysis of single-cell sequencing results in Example 1, in which (a) shows read length distribution, (b) shows distribution of the number of reads for different cells, and (c) shows single-cell purity distribution.
Fig. 9 shows statistics of single-cell genome coverage in Example 1.
Fig. 10 is a schematic diagram showing droplet formation by pipetting to encapsulate hydrogel beads for whole-genome amplification.
Fig. 11 shows a comparison of single-cell purity distribution after whole-genome amplification performed in encapsulated hydrogel microchambers and in permeable hydrogel microchambers in an aqueous solution.
Fig. 12 shows a comparison between exponential amplification and linear amplification in terms of single-cell purity distribution and indexing efficiency.
Fig. 13 shows sequencing results of a ligation-based method, in which (a) shows read length distribution, (b) shows distribution of the number of reads for different cells, and (c) shows single-cell purity distribution.

### DETAILED DESCRIPTION OF THE PREFERRED EMBODIMENTS

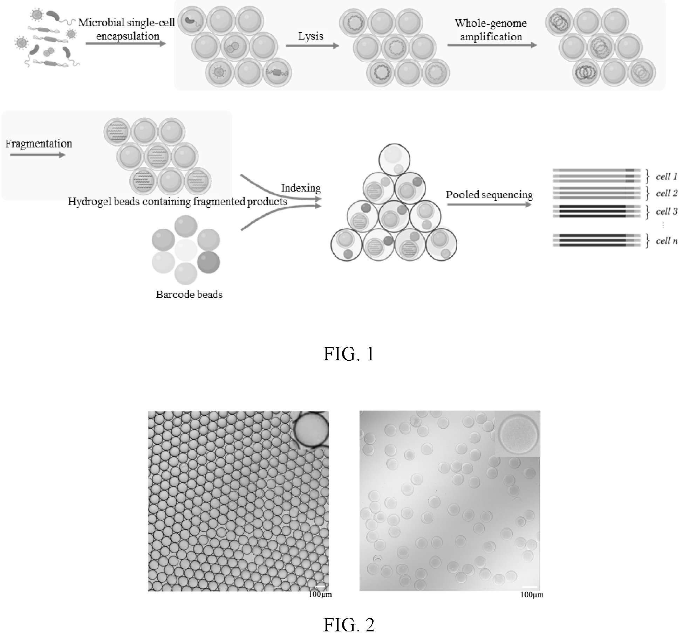

To simplify the library preparation workflow, improve microbial single-cell purity and genome coverage, and reduce time, cost, and labor, as shown in Fig. 1, the present disclosure provides a method for constructing a microbial single-cell whole-genome sequencing library, which includes the following steps:
1) providing microchambers each containing a single microbial cell, lysing the single cell, and amplifying a whole genome of the single cell, thereby obtaining microchambers each containing a single-cell whole genome;
2) fragmenting the whole genome obtained in step 1), thereby obtaining microchambers each containing a fragmented genome;
3) introducing a barcode bead into each of the microchambers obtained in step 2) and barcoding the fragmented genome, thereby obtaining microchambers each containing a barcoded whole genome; and
4) dissolving the microchambers obtained in step 3), and performing enrichment amplification of the barcoded whole genome, thereby obtaining a microbial single-cell whole-genome sequencing library.

In some embodiments, the microorganism may be any one of bacteria, viruses, fungi, actinomycetes, rickettsiae, mycoplasmas, chlamydiae, and spirochetes.

In some embodiments, the bacteria may be culturable bacteria or nonculturable bacteria. Preferably, the culturable bacteria include, but are not limited to, *Escherichia coli*, *Bacillus subtilis*, *Staphylococcus aureus*, and/or *Pseudomonas aeruginosa*.

In some embodiments, the microchambers may be gel beads or gel droplets (also referred to as microdroplets). The gel droplets are microdroplets containing a gel material (e.g., a hydrogel), which are generated by droplet microfluidic technology, a pipetting method, a vortexing method, a membrane emulsification method, an electric driving method, an inkjet printing method, or the like. The microdroplets are converted into gel beads after demulsification.

In the present disclosure, the reactions carried out in microchambers during the library construction may be performed entirely using a microfluidic chip or only partially using a microfluidic chip. When the microfluidic chip is used only for part of the process, microchamber preparation, barcode bead preparation, and DNA indexing need to be completed in the microfluidic chip, and these reactions take place in gel droplets. In addition, whole-genome amplification also needs to be completed in gel droplets, which may be generated either by a microfluidic chip or by pipetting, vortexing, or the like, as long as the whole-genome amplification is carried out in gel droplets. Other microchamber-related steps, such as lysis and fragmentation, may be performed by placing gel beads in a reaction system. In this way, on the one hand, the operational difficulty and time can be significantly reduced, and on the other hand, steps requiring microfluidics are reduced, thereby avoiding cross-contamination in an open environment.

Illustratively, the present disclosure provides a method for constructing a microbial single-cell whole-genome sequencing library, which includes the following steps:
S1) using a microfluidic chip to capture microbial single cells, thereby providing microdroplets each containing a single microbial cell, demulsifying the microdroplets to form gel beads each containing a single microbial cell, lysing the single microbial cell in each gel bead, and then re-forming microdroplets from the gel beads by a pipetting method or a vortexing method, followed by performing whole-genome amplification of the single cell in each microdroplet, thereby obtaining microdroplets each containing a single-cell whole genome;
S2) demulsifying the obtained microdroplets each containing a single-cell whole genome to obtain gel beads each containing a single-cell whole genome, and then fragmenting the whole genome in each gel bead, thereby obtaining gel beads each containing a fragmented genome;
S3) using a microfluidic chip to introduce a barcode bead into each of the gel beads obtained in step S2), and barcoding the fragmented genome, thereby obtaining microdroplets each containing a barcoded whole genome; and
S4) dissolving the microdroplets obtained in step S3), such that the barcoded whole genomes are transferred into an aqueous solution, and then performing enrichment amplification of the barcoded whole genomes, thereby obtaining a microbial single-cell whole-genome sequencing library.

In some embodiments, the demulsification method mainly includes a chemical method and a physical method. The chemical method includes demulsifying the microdroplets by using a demulsifier, acidification, or alkalization, wherein the demulsifier includes, but is not limited to, a Drop-Surf demulsifier, a PFO demulsifier, and a Drop-Break demulsifier. The physical method includes, but is not limited to, centrifugation and agitation.

In some embodiments, the gel contains a gel polymer.

In some embodiments, the gel polymer includes one or more of a dextran gel, a poly(ethylene glycol) diacrylate gel, a polyacrylamide gel, an agarose gel, a gelatin methacryloyl gel, four-arm polyethylene glycol (4-arm PEG), eight-arm polyethylene glycol (8-arm PEG), polyethylene glycol-maleimide (PEG-maleimide), and thiol-polyethylene glycol-thiol (thiol-PEG-thiol); preferably, two thereof are included.

The present disclosure can capture a single cell in a gel droplet by using a high-throughput microdroplet technology. Under suitable conditions, including but not limited to UV irradiation, a temperature change, and a pH change, the gel droplet containing the single cell can be solidified into a gel bead containing the single cell.

In some embodiments, the operation for lysing the single cell includes, but is not limited to, the following methods or a combination thereof:
(A1) an enzymatic lysis method, i.e., using a specific enzyme to perform a corresponding chemical reaction to lyse the cell and release intracellular DNA;
(B1) an alkaline lysis method, i.e., under a specific alkaline condition (e.g., pH > 7 and pH ≤ 14), together with a suitable temperature, lysing the cell and releasing intracellular DNA; and
(C1) a heat-shock method, i.e., repeatedly freezing and thawing the cell to cause cell swelling, thereby disrupting the cell structure and releasing intracellular DNA.

For reactions such as PCR, the lysis step may be omitted because the denaturation temperature of the PCR reaction can lyse the cell and release intracellular DNA.

In some embodiments, the lysis is in situ lysis, for example, direct lysis in the microchamber.

In some embodiments, enzymes used in the enzymatic lysis method may include one or more of Labiase lysing enzyme, lysostaphin, lysozyme, mutanolysin, zymolyase, and proteinase K.

In some embodiments, reagents for lysing the single cell include a lysis buffer A and a lysis buffer B.

In some embodiments, the lysis buffer A includes lysostaphin, mutanolysin, lysozyme, and zymolyase.

In some embodiments, the working concentration of lysostaphin is in a range of 30-50 U/mL; for example, 30-40 U/mL or 40-50 U/mL; specifically, 30 U/mL, 35 U/mL, 40 U/mL, 45 U/mL, or 50 U/mL.

In some embodiments, the working concentration of mutanolysin is in a range of 80-120 U/mL; for example, 80-90 U/mL, 90-100 U/mL, 100-110 U/mL, or 110-120 U/mL; specifically, 80 U/mL, 85 U/mL, 90 U/mL, 95 U/mL, 100 U/mL, 105 U/mL, 110 U/mL, 115 U/mL, or 120 U/mL.

In some embodiments, the working concentration of lysozyme is in a range of 0.5-4 U/µL; for example, 0.5-1.8 U/µL, 1.8-3 U/µL, or 3-4 U/µL; specifically, 0.5 U/µL, 1 U/µL, 1.5 U/µL, 1.8 U/µL, 2 U/µL, 2.5 U/µL, 3 U/µL, 3.5 U/µL, or 4 U/µL.

In some embodiments, the working concentration of zymolyase is in a range of 0.005-0.02 U/µL; for example, 0.005-0.01 U/µL or 0.01-0.02 U/µL; specifically, 0.005 U/µL, 0.008 U/µL, 0.01 U/µL, 0.015 U/µL, or 0.02 U/µL.

In some embodiments, the lysis buffer A further includes one or more of NaCl, EDTA, Tris-HCl, and Triton X-100.

In some embodiments, the working concentration of NaCl in the lysis buffer A is in a range of 30-70 mM; for example, 30-40 mM, 40-50 mM, 50-60 mM, or 60-70 mM; specifically, 30 mM, 40 mM, 45 mM, 50 mM, 55 mM, 60 mM, or 70 mM.

In some embodiments, the working concentration of EDTA in the lysis buffer A is in a range of 3-8 mM; for example, 3-5 mM or 5-8 mM; specifically, 3 mM, 4 mM, 5 mM, 6 mM, 7 mM, or 8 mM.

In some embodiments, the working concentration of Tris-HCl in the lysis buffer A is in a range of 5-15 mM; for example, 5-8 mM, 8-11 mM, or 11-15 mM; specifically, 5 mM, 6 mM, 7 mM, 8 mM, 9 mM, 10 mM, 11 mM, 12 mM, 13 mM, 14 mM, or 15 mM. Preferably, the pH of the Tris-HCl is in a range of 7.5-8.5.

In some embodiments, the working concentration of Triton X-100 in the lysis buffer A is in a range of 0.1-1 v/v%; for example, 0.1-0.3 v/v%, 0.3-0.6 v/v%, or 0.6-1 v/v%; specifically, 0.4 v/v%, 0.5 v/v%, 0.6 v/v%, or 0.7 v/v%.

In some embodiments, the volume ratio of the lysis buffer A to the microchambers is in a range of 1:0.8-1.2; for example, 1:0.8-1 or 1:1-1.2; preferably, 1:1.

In some embodiments, the lysis buffer B includes proteinase K.

In some embodiments, the lysis buffer B further includes one or more of NaCl, EDTA, Tris-HCl, and SDS.

In some embodiments, the working concentration of proteinase K is in a range of 0.1-0.5 mg/mL; for example, 0.1-0.3 mg/mL or 0.3-0.5 mg/mL; specifically, 0.1 mg/mL, 0.2 mg/mL, 0.3 mg/mL, 0.4 mg/mL, or 0.5 mg/mL.

In some embodiments, the working concentration of NaCl in the lysis buffer B is in a range of 80-120 mM; for example, 80-100 mM or 100-120 mM; specifically, 80 mM, 90 mM, 100 mM, 110 mM, or 120 mM.

In some embodiments, the working concentration of EDTA in the lysis buffer B is in a range of 5-15 mM; for example, 5-10 mM or 10-15 mM; specifically, 5 mM, 8 mM, 10 mM, 12 mM, or 15 mM.

In some embodiments, the working concentration of Tris-HCl in the lysis buffer B is in a range of 5-15 mM; for example, 5-8 mM, 8-11 mM, or 11-15 mM; specifically, 5 mM, 6 mM, 7 mM, 8 mM, 9 mM, 10 mM, 11 mM, 12 mM, 13 mM, 14 mM, or 15 mM. Preferably, the pH of the Tris-HCl is in a range of 7.5-8.5.

In some embodiments, the working concentration of SDS in the lysis buffer B is in a range of 0.01-2 w/v%; for example, 0.01-0.5 w/v%, 0.5-1 w/v%, or 1-2 w/v%; specifically, 0.01 w/v%, 0.5 w/v%, 1 w/v%, 1.5 w/v%, or 2 w/v%.

In some embodiments, the working concentration of proteinase K is in a range of 0.1-0.5 mg/mL; for example, 0.1-0.3 mg/mL or 0.3-0.5 mg/mL; specifically, 0.1 mg/mL, 0.2 mg/mL, 0.3 mg/mL, 0.4 mg/mL, or 0.5 mg/mL.

In some embodiments, the volume ratio of the lysis buffer B to the microchambers is in a range of 1:0.8-1.2; for example, 1:0.8-1 or 1:1-1.2; preferably, 1:1.

In some embodiments, the lysis procedure includes mixing the lysis buffer A with the microchambers to perform the first lysis, washing the microchambers after the first lysis, and then adding the lysis buffer B to perform the second lysis.

In some embodiments, the temperature of the first lysis is in a range of 30-43°C; for example, 30-35°C, 35-40°C, or 40-43°C; specifically, 33°C, 37°C, or 40°C.

In some embodiments, the rotational speed of the first lysis is in a range of 1300-1800 rpm; for example, 1300-1400 rpm, 1400-1500 rpm, 1500-1600 rpm, or 1600-1800 rpm; specifically, 1400 rpm, 1500 rpm, or 1600 rpm.

In some embodiments, the duration of the first lysis is in a range of 8-16 h; for example, 8-10 h, 10-13 h, or 13-16 h; specifically, 8 h, 10 h, 12 h, or 14 h.

In some embodiments, the temperature of the second lysis is in a range of 50-65°C; for example, 50-55°C or 55-60°C; specifically, 53°C, 55°C, 58°C, or 60°C.

In some embodiments, the rotational speed of the second lysis is in a range of 1300-1800 rpm; for example, 1300-1400 rpm, 1400-1500 rpm, 1500-1600 rpm, or 1600-1800 rpm; specifically, 1400 rpm, 1500 rpm, or 1600 rpm.

In some embodiments, the duration of the second lysis is in a range of 0.5-2 h; for example, 0.5-1 h, 1-1.5 h, or 1.5-2 h; specifically, 0.5 h, 1 h, or 1.5 h.

In some embodiments, each microchamber includes an inner layer and an outer layer, wherein the inner layer contains a hydrogel of a first polymer, and the outer layer contains a hydrogel of a second polymer. Such a structure enables the microchamber to be a permeable microchamber, i.e., macromolecules are retained in the microchamber while small molecules can enter and exit, such that microbial single-cell lysis and DNA release, amplification, fragmentation, and DNA indexing, including but not limited to PCR- or ligation-based DNA indexing, can be achieved in the microchamber. Reagents used herein for lysis, PCR, DNA indexing, and the like are all capable of entering the microchamber.

In some embodiments, the first polymer and the second polymer are each independently selected from dextran, high-molecular-weight poly(ethylene glycol) diacrylate, polyacrylamide gel, agarose gel, gelatin methacryloyl gel, 4-arm PEG, 8-arm PEG, PEG-maleimide, and thiol-PEG-thiol, wherein the first polymer is different from the second polymer.

In some preferred embodiments, the first polymer is dextran, and the second polymer is poly(ethylene glycol) diacrylate (PEGDA).

In some specific embodiments, raw materials for preparing the microchambers include dextran and high-molecular-weight poly(ethylene glycol) diacrylate.

In some embodiments, the weight-average molecular weight of the dextran is in a range of 10k-2000k; for example, 10k-100k, 100k-500k, 500k-1000k, 1000k-1500k, or 1500k-2000k.

In some preferred embodiments, the weight-average molecular weight of the high-molecular-weight poly(ethylene glycol) diacrylate is in a range of 1K-20K; for example, 1K-5K, 5K-10K, 10K-15K, or 15K-20K; wherein the high-molecular-weight poly(ethylene glycol) diacrylate is typically in a solid or waxy semi-solid form.

In some embodiments, a solvent for the dextran and the poly(ethylene glycol) diacrylate is a mixture of a DPBS solution of low-molecular-weight poly(ethylene glycol) diacrylate and an aqueous solution of lithium phenyl(2,4,6-trimethylbenzoyl)phosphinate.

In some embodiments, the weight-average molecular weight of the low-molecular-weight poly(ethylene glycol) diacrylate is in a range of 200-900; specifically, 200, 250, 400, 575, 600, 700, 800, or 900, and the low-molecular-weight poly(ethylene glycol) diacrylate is typically in a liquid or viscous liquid form.

In some embodiments, in the solvent, the volume ratio of the DPBS solution of low-molecular-weight poly(ethylene glycol) diacrylate to the aqueous solution of lithium phenyl(2,4,6-trimethylbenzoyl)phosphinate is in a range of 60-80:8-12; for example, 65-75:9-11; specifically, 73:10.

In some embodiments, the volume ratio of poly(ethylene glycol) diacrylate (MW 575) to 1× DPBS is in a range of 2-4:60-80 (v/v); specifically, 3:70 (v/v).

In some embodiments, the concentration of lithium phenyl(2,4,6-trimethylbenzoyl)phosphinate in the aqueous solution is in a range of 0.02-0.06 g/mL; for example, 0.03-0.05 g/mL; specifically, 0.02 g/mL, 0.03 g/mL, 0.04 g/mL, 0.05 g/mL, or 0.06 g/mL.

In some embodiments, the concentration of dextran in the raw materials for preparing the microchambers is in a range of 40-75 mg/mL; for example, 55-70 mg/mL; specifically, 40 mg/mL, 45 mg/mL, 50 mg/mL, 55 mg/mL, 60 mg/mL, 66.3 mg/mL, 70 mg/mL, or 75 mg/mL.

In some embodiments, the concentration of the high-molecular-weight poly(ethylene glycol) diacrylate in the raw materials for preparing the microchambers is in a range of 20-50 mg/mL; for example, 25-45 mg/mL; specifically, 20 mg/mL, 25 mg/mL, 30 mg/mL, 36.1 mg/mL, 40 mg/mL, 45 mg/mL, or 50 mg/mL.

In some embodiments, amplification of the whole genome of the single cell in step 1) and the enrichment amplification in step 4) include, but are not limited to, exponential amplification or linear amplification. For example, the amplification method may be one of the following:
(A2) polymerase chain reaction (PCR), in which whole-genome amplification of a single cell is performed using a polymerase chain reaction;
(B2) rolling circle amplification (RCA), which is rolling circle amplification using a short circular single-stranded DNA as a template;
(C2) multiple annealing and looping-based amplification cycles (MALBAC), in which the single-cell genome is subjected to multiple annealing and looping-based amplification cycles;
(D2) multiple displacement amplification (MDA), in which whole-genome amplification is performed by binding random primers and a polymerase, including but not limited to phi29 DNA polymerase, to a DNA template; and
(E2) linear amplification via transposon insertion (LIANTI), in which linear amplification of the single-cell genome is performed through transposon insertion.

In single-cell whole-genome amplification, either exponential amplification or linear amplification may be used, as long as whole-genome amplification is achieved. Meanwhile, during the whole-genome DNA reaction process, additional inducers may be added to improve reaction efficiency, wherein the inducers include, but are not limited to, pyrophosphatase and the like. When in situ whole-genome amplification is performed on single cells, reactants of the amplification system are introduced into the permeable microchambers, followed by whole-genome amplification.

The term "fragment" as used herein refers to any nucleic acid sequence that is shorter than the sequence from which it is derived. A fragment may be of any size, ranging from several megabases and/or kilobases to several nucleotides in length.

The term "fragmentation" as used herein refers to any process or method for breaking a compound or composition into smaller units. For example, the separation may include, but is not limited to, enzymatic cleavage (for example, transposase-mediated fragmentation, a restriction enzyme acting on a nucleic acid, or a protease acting on a protein), alkaline hydrolysis, acid hydrolysis, or heatinduced destabilization.

In some embodiments, genome fragmentation may be performed using an enzymatic fragmentation method or a non-enzymatic fragmentation method.

In some embodiments, the non-enzymatic fragmentation method includes, but is not limited to, ultrasonic fragmentation, high-pressure shearing, alkaline hydrolysis or acid hydrolysis fragmentation, and G-tube shearing.

In some embodiments, preferably, the enzymatic fragmentation method uses dsDNA Fragmentase, T7 Endonuclease I, DNase I, or a transposase.

In some embodiments, the single-cell whole genome may be fragmented using a Tn5 transposome complex. The Tn5 transposome complex is composed of a Tn5 transposase protein and a specific DNA sequence (such as a Mosaic End (ME) sequence), which are combined together to form an active transposome complex. This complex has a particular steric structure and activity, enabling it to cleave a target DNA sequence and add a DNA sequence to both ends of the fragmented genomic DNA, which can serve as primer binding sites for ligating barcode sequences to the fragmented genomic DNA.

In some embodiments, each barcode bead carries copies of a unique barcode sequence. In preparing the barcode bead, it is first necessary to design 96 × 96 × 96 oligonucleotide sequences of 6-9nt as a barcode tag library, and to synthesize three sets of 96 oligonucleotide sequences in three 96-well plates. During the first round of nucleic acid labeling, the permeable microchambers containing fragmented microbial single-cell whole-genome amplification products are evenly and randomly distributed into a 96-well plate, which contains reagents required for a PCR reaction. Through the PCR reaction, a first round of barcode sequence addition to the microbial single-cell genome is achieved. After the reaction is completed, the permeable microchambers are collected from the 96-well plate for that round, mixed, and washed to remove excess oligonucleotide sequences that have not been ligated or have not been added to the genome by PCR, as well as enzymes or metal ions introduced during the reaction process. After the first round of nucleic acid labeling, the washed permeable microchambers are again evenly and randomly distributed into a 96-well plate, and a PCR reaction is performed to achieve addition of a second round of barcode sequences. After the reaction is completed, the permeable microchambers may be collected from the 96-well plate for that round and mixed, followed by a third round of nucleic acid labeling. After repeating the above steps of even random distribution, PCR reaction, mixing, and washing, the fragmented microbial whole-genome DNA in each obtained permeable microchamber carries a unique barcode tag formed by free combination through the three rounds.

The barcode sequence formed by free combination through the three rounds includes: Barcode1-fixed structure-Barcode2-fixed structure-Barcode3. The barcode sequence includes alternately arranged fixed structures and variable structures (Barcode1, Barcode2, and Barcode3). For example, the fixed structure of the barcode sequence may be selected from ACAG, GTCA, TGCC, CCGCT, GTCT, and GTCT. The fixed structures are used in subsequent analysis to anchor the variable structures of the barcode sequence and facilitate the extraction of the variable structures of the barcode sequence for single-cell analysis. The variable structure sequence of the barcode sequence is NNNNNNN, and the number of Ns in the sequence depends on the number of single cells to be analyzed, so as to ensure that one barcode sequence corresponds to one and only one single cell. If the number of single cells is large, the number of Ns may be increased, for example, NNNNNN, NNNNNNN, NNNNNNNN, NNNNNNNNN, and the like, wherein N is A, G, C, or T.

In some embodiments, the barcode sequence further includes a 5' adapter and a 3' adapter, wherein the 5' adapter and the 3' adapter contain primer binding sites for subsequent PCR amplification or sequencing.

In some embodiments, the barcode sequence is

TTTCTACACGACGCTCTTCCGATCTNNNNNNNACAGNNNNNNNGTCAN
NNNNNNGTCTCGTGGGCTCGG (SEQ ID NO. 2).

In some embodiments, the barcode bead includes at least one of a polyacrylamide bead, a polystyrene bead, a poly(ethylene glycol) diacrylate bead, a poly(lactic-co-glycolic acid) bead, an agarose bead, and a magnetic bead.

In some embodiments, in step 3), barcoding the fragmented genome is performed by ligation using a ligase or by single-primer amplification. For example, oligonucleotides carrying a barcode may be ligated to a genomic fragment by a DNA ligase; alternatively, a barcode sequence may be introduced into a genomic fragment by single-primer amplification, thereby barcoding the fragmented genome and completing genome indexing.

In the prior art, the barcode sequence may also be introduced into a genomic fragment by a conventional PCR amplification method (e.g., exponential amplification), thereby barcoding the fragmented genome. However, due to the particular structure of the permeable microchambers, the inventors of the present disclosure have found that exponential amplification is not suitable. After whole-genome amplification, the confined space of each microchamber is already filled with nucleic acid molecules, and thus it is unnecessary to further increase the amount of nucleic acid molecules by exponential amplification. On the contrary, if exponential amplification is performed in the restricted space where both forward and reverse primers are present, primer dimers are more likely to form, thereby generating non-specific amplification products. Accordingly, the present disclosure innovatively adopts single-primer amplification, i.e., linear amplification, to achieve single-cell indexing. Only one primer at one end is required, thereby avoiding non-specific amplification.

When a barcode sequence is introduced into a fragment by linear amplification, one barcode bead and one permeable microchamber containing fragmented single-cell genomic DNA are co-encapsulated in one droplet, and the droplet is then placed in a PCR instrument for amplification.

In some embodiments, the linear amplification solution includes a DNA polymerase, a buffer, ET-SSB, dNTPs, or other PCR or ligation reaction reagents.

In some embodiments, the DNA polymerase includes, but is not limited to, any one of, or a combination of two or more of, Bst 2.0 WarmStart DNA polymerase, Platinum DNA polymerase, Vent DNA polymerase, T7 DNA polymerase, T4 DNA polymerase, DNA polymerase I, Sulfolobus DNA polymerase IV, phi29 DNA polymerase, Bst DNA polymerase, EquiPhi29 DNA polymerase, Deep Vent DNA Polymerase, and Phusion High-Fidelity DNA Polymerase.

In some embodiments, when barcoding the fragmented genome, the method further includes a step of releasing the barcode from the barcode bead, including but not limited to releasing the barcode from the barcode bead by chemical bond cleavage (e.g., cleavage of a disulfide bond), enzymatic degradation, or ultraviolet cleavage. For example, DTT (dithiothreitol), a reagent capable of cleaving disulfide bonds, may be added to the linear amplification solution to release the barcode into the microdroplet for barcoding the fragmented genome. Alternatively, dichloromethane, ethyl acetate, acetone, PBS, a specific degradation enzyme, light of a specific wavelength, or the like may be used. A corresponding method may be selected according to the material of the barcode bead, with the main purpose of dissolving the barcode bead and releasing the barcode sequence therein.

In some embodiments, the PCR solution used for linear amplification includes: 100-140 µL of Phusion High-Fidelity Reaction Buffer, 10-20 µL of Phusion High-Fidelity DNA Polymerase, 10-20 µL of Deep Vent DNA Polymerase, 10-15 µL of ET-SSB, 60-70 µL of 10× PCR stabilizer, 60-70 µL of 8-12 mM dNTPs, and water added to a final volume of 340-360 µL.

In some embodiments, the PCR solution used for linear amplification further includes 60-70 µL of 80-120 mM DTT.

In some embodiments, the primer for linear amplification is a single primer sequence, which may be located on the barcode bead or added together with the PCR reaction system, without particular limitation. Illustratively, in the present disclosure, the primer sequence is located on the barcode bead.

In some embodiments, the reaction conditions for the linear amplification include initial denaturation, a cycling stage (denaturation, annealing, and extension), and final extension.

In some preferred embodiments, the conditions for the linear amplification are: 70-74°C for 4-6 min; 96-99°C for 2-4 min; 96-99°C for 8-12 s, 58-62°C for 28-32 s, and 70-74°C for 4-6 min per cycle, with 14-16 cycles; and holding at 3-6°C.

In some embodiments, when barcoding the fragmented genome by a DNA ligase, the fragmented single-cell genomic sequence is ligated to the barcode sequence by the DNA ligase.

In some embodiments, the DNA ligase is T7 DNA ligase, T4 DNA ligase, E. coli DNA ligase, or the like.

In some embodiments, when a ligase-based method is used to barcode the fragmented genome, the DNA is typically first subjected to fragmentation treatment (e.g., enzymatic digestion or mechanical shearing). When a Tn5 transposome complex is used for fragmentation treatment, a phosphorylated primer may be used in preparing the Tn5 transposome complex. During the final round of barcode synthesis, the primer used therein may also be phosphorylated in preparing the barcode bead, thereby further improving primer stability and ligation efficiency.

In some embodiments, in step 3), the barcode bead and the microchamber containing the fragmented genomic product are co-encapsulated in a droplet, and a ligation reaction is performed, thereby achieving indexing of the genomic DNA in the permeable microchamber.

In some embodiments, the method for dissolving the microchambers obtained in step 4) includes, but is not limited to, a chemical dissolution method, a physical method, and electric field- or magnetic field-assisted methods. The chemical dissolution method specifically refers to introducing a chemical reagent into the microfluidic chip, the components in the microdroplets undergo a chemical reaction, thereby achieving dissolution. For example, a strongly alkaline solution such as sodium hydroxide may be used to dissolve certain organic-phase microdroplets. In addition, dissolution of the microdroplets may be accelerated by adjusting the polarity or pH of the solvent.

In some embodiments, before performing the enrichment amplification in step 4), the method further includes purifying the barcoded whole genome. The purification method includes, but is not limited to, using a commercial purification kit, such as a column purification reagent, a magnetic bead purification reagent, or a gel electrophoresis reagent.

In some embodiments, the enrichment amplification includes, but is not limited to, exponential amplification or linear amplification; preferably, the enrichment amplification is exponential amplification. Exponential amplification can generate a large amount of target nucleic acid within a short period of time, thereby further enriching the linear amplification product. At this stage, the DNA subjected to enrichment amplification is in an aqueous solution rather than in a confined space. In addition, a higher yield of nucleic acid is required to meet sequencing requirements, because sequencing generally requires a relatively large amount of DNA template. For example, a single sequencing run generally requires 400 ng of DNA, although the amount of DNA template may be adjusted according to the sequencing platform.

In some embodiments, the reaction system for the exponential amplification includes a DNA template, a forward primer, a reverse primer, a DNA polymerase, a dNTP mixture, a buffer, and other PCR amplification reagents.

In some embodiments, after the enrichment amplification, the method further includes ligating DNA fragments to adapter sequences. The adapter sequences include, but are not limited to, universal Read 1 and Read 2 adapter sequences for the Nextera sequencing platform, universal P5 and P7 adapter sequences for the Illumina sequencing platform, and self-designed adapter sequences. After addition of the adapter sequences is completed, the sequencing library may further be subjected to size selection and library purification. The selection methods include, but are not limited to, purification columns, magnetic beads, and gel electrophoresis. After library construction and sequencing, metagenomic analysis capable of identifying individual genomes may be performed.

In some embodiments, the sequencing adapter may be a next-generation sequencing adapter or a third-generation sequencing adapter.

The present disclosure further provides a method for single-cell genome sequencing, wherein the method includes:
a) constructing a sequencing library according to the method for constructing the sequencing library described above; and
b) sequencing the sequencing library provided in step a).

In some embodiments, the sequencing method may be next-generation sequencing or third-generation sequencing.

The present disclosure further provides a product for constructing a microbial single-cell whole-genome sequencing library, wherein the product includes barcode beads suitable for the above sequencing library construction, single-cell whole-genome amplification reagents, indexing reagents, and enrichment amplification reactants.

In some embodiments, the indexing reagents include linear amplification reagents or a DNA ligase.

In some embodiments, the product further includes a lysis buffer, fragmentation-related reagents, raw materials for preparing microchambers, and the like.

In some embodiments, the product may be a kit, a chip, or a detection system; preferably, the chip is a microfluidic chip.

In some embodiments, the single cell is a microbial single cell.

In some embodiments, the microorganism is a single strain or a mixed strain; the mixed strain includes a mixture of two or more strains.

Specific embodiments of the present disclosure are described below. Those skilled in the art can readily appreciate other advantages and effects of the present disclosure from the disclosure of this specification.

Before the specific embodiments of the present disclosure are further described, it should be understood that the protection scope of the present disclosure is not limited to the specific embodiments set forth below. It should also be understood that the terminology used in the examples of the present disclosure is for the purpose of describing specific embodiments only, and is not intended to limit the protection scope of the present disclosure. For the experimental methods in the following examples for which specific conditions are not indicated, the methods are generally carried out under conventional conditions or under the conditions recommended by the respective manufacturers.

When a numerical range is given in an example, it should be understood that, unless otherwise indicated in the present disclosure, both endpoints of the numerical range and any value between the two endpoints may be selected. Unless otherwise defined, all technical and scientific terms used herein have the same meanings as commonly understood by those skilled in the art. In addition to the specific methods, devices, and materials used in the examples, any methods, devices, and materials of the prior art that are similar or equivalent to those described in the examples of the present disclosure may also be used to implement the present disclosure, based on the knowledge of those skilled in the art and the description of the present disclosure.

In the present disclosure, the unit w/v refers to a mass-to-volume ratio, wherein the unit of mass is g and the unit of volume is mL, expressed as grams per milliliter (g/mL); for example, 1 w/v% means that 100 mL of solution contains 1 g of a substance.

In the present disclosure, unless otherwise specifically indicated, all nucleic acid sequences involved are described by default in the direction from the 5' end to the 3' end.

### Examples 1-3

In these examples, microbial single-cell whole-genome amplification, indexing, and library construction were performed on an artificial mixed bacterial sample prepared by mixing *Escherichia coli* and *Bacillus subtilis* at an equal ratio. The procedure specifically included the following steps:

The required solutions were prepared according to the following formulation.

700 µL of DPBS, 0.055 g of Dextran, 0.03 g of PEGDA (8K), 30 µL of PEGDA (575), and 100 µL of 4 w/v% LAP (lithium phenyl(2,4,6-trimethylbenzoyl)phosphinate) were added to a centrifuge tube. The mixture was dissolved, mixed, and centrifuged (16,000 ×g, 30 min, room temperature). Phase separation then occurred, and the upper PEGDA-rich phase and the lower Dextran-rich phase were separately aspirated into two new 1.5 mL centrifuge tubes.

*Escherichia coli* and *Bacillus subtilis* were separately cultured in Luria-Bertani (LB) medium at 37°C for 12 h. Then, 10 µL of each bacterial culture was transferred into fresh LB medium and cultured at 37°C for 16 h. Thereafter, 1 mL of the *Escherichia coli* culture and 1 mL of the *Bacillus subtilis* culture were separately collected into 1.5 mL centrifuge tubes, centrifuged at 5000 rpm for 2 min to remove the supernatant, washed with DPBS buffer, and centrifuged again to remove the supernatant. The bacterial pellets were resuspended in DPBS buffer. 200 µL of each bacterial suspension was transferred into a 96-well plate, and the absorbance at 600 nm was measured using a microplate reader. The numbers of *Escherichia coli* and *Bacillus subtilis* were separately calculated based on the relationship between bacterial concentration and absorbance (OD600 = 0.93, corresponding to 9.3 × 10⁸ CFU/mL). Equal numbers of the two bacteria were added to 200 µL of the Dextran-rich phase and mixed well to give a final concentration of 2000 bacteria/µL.

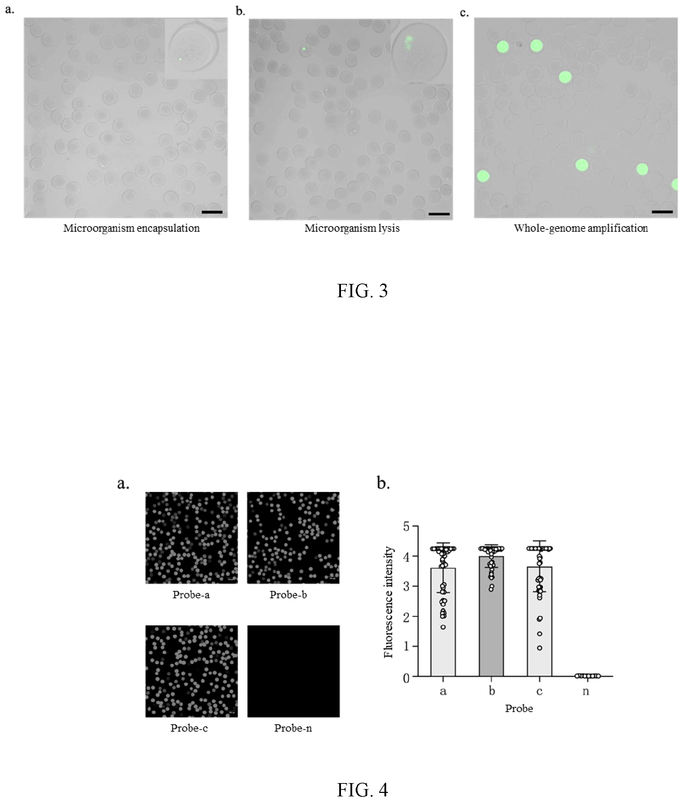

A microfluidic droplet-generation chip was used to introduce the Dextran-rich phase containing the mixed bacterial suspension of *Escherichia coli* and *Bacillus subtilis* and the PEGDA-rich phase not containing the mixed bacterial suspension of *Escherichia coli* and *Bacillus subtilis* into the corresponding channels of the chip. Using high-throughput microdroplet technology, microdroplets encapsulating microbial single cells were generated, wherein the microdroplets were completely enclosed microchambers. Each microdroplet included an outer PEGDA-rich layer and an inner Dextran-rich layer, and the inner layer contained the liquid medium of the mixed bacterial suspension, while each microdroplet encapsulated a single microbial cell from the mixed bacterial suspension. The microdroplets were collected in a sterile centrifuge tube, irradiated under ultraviolet light at a wavelength of 365-370 nm for 2 min, and then demulsified using PFO. After demulsification, the microdroplets formed hydrogel beads, which were permeable microchambers, also referred to as hydrogel microchambers. Microscopic images of the microdroplets and the hydrogel microchambers are shown in Fig. 2. The permeable microchambers containing microbial single cells were collected after being washed three times with DPBS buffer containing 0.1 v/v% Tween-20. 9.5 µL thereof was mixed with 0.5 µL of SYBR Green solution, observed under a fluorescence microscope, and fluorescence images under excitation light at 488 nm were captured (Fig. 3a).

Microbial single cells in the permeable hydrogel microchambers were lysed by a stepwise enzymatic lysis method. Lysis buffers A and B were prepared according to Tables 1 and 2, respectively. First, 500 µL of the hydrogel microchambers each encapsulating a microbial single cell was transferred into a new 1.5 mL centrifuge tube, and 500 µL of lysis buffer A was added. The mixture was subjected to an overnight lysis reaction in a thermostatic mixer at 37°C and 1500 rpm, thereby completing the first lysis reaction. Subsequently, the hydrogel microchambers after the first lysis were washed with DPBS buffer containing 0.1% (v/v) Tween, and then 500 µL of lysis buffer B was added. The mixture was cultured in a thermostatic mixer at 55°C and 1500 rpm for 1 h, thereby lysing the microbial single cells in the hydrogel microchambers. The hydrogel microchambers were then washed multiple times with Tris-EDTA buffer (pH 8.0) containing 0.1% (v/v) Tween and stored at 4°C for subsequent amplification reactions. Thereafter, 9.5 µL of the hydrogel microchambers was mixed with 0.5 µL of SYBR Green solution, observed under a fluorescence microscope, and fluorescence images under excitation light at 488 nm were captured (Fig. 3b).

**Table 1 Lysis buffer A for hydrogel microchambers each encapsulating a microbial single cell**

| Example No. | Example 1 | Example 2 | Example 3 |
|---|---|---|---|
| Component | Working concentration | | |
| Nuclease-Free Water | bring to 500µL | bring to 500µL | bring to 500µL |
| 1M NaCl | 50 mM | 30 mM | 70 mM |
| 100 mM EDTA | 5 mM | 3 mM | 8 mM |
| 200 mM Tris-HCl (pH 8.0) | 10mM | 15 mM | 5 mM |
| 10v/v% Triton X-100 | 0.5v/v% | 0.1 v/v% | 1 v/v% |
| 500 U/mL Lysostaphin | 40 U/mL | 30 U/mL | 50 U/mL |
| 2500 U/mL Mutanolysin | 100 U/mL | 120 U/mL | 80 U/mL |
| 300 U/µL Ready-Lyse^{™} Lysozyme Solution | 1.8 U/µL | 0.5 U/µL | 4 U/µL |
| 2 U/µL Zymolyase | 0.008 U/µL | 0.005 U/µL | 0.02 U/µL |

**Table 2 Lysis buffer B for hydrogel microchambers each encapsulating a microbial single cell**

| | Example 1 | Example 2 | Example 3 |
|---|---|---|---|
| Component | Working concentration | | |
| Nuclease-Free Water | bring to 500µL | bring to 500µL | bring to 500µL |
| 1M NaCl | 100 mM | 80 mM | 120 mM |
| 100 mM EDTA | 10 mM | 5 mM | 15 mM |
| 200 mM Tris-HCl (pH 8.0) | 10 mM | 15 mM | 5 mM |
| 10 w/v% SDS | 0.5w/v% | 0.01 w/v% | 2 w/v% |
| 20 mg/mL proteinase K | 0.2 mg/mL | 0.5 mg/mL | 0.1 mg/mL |

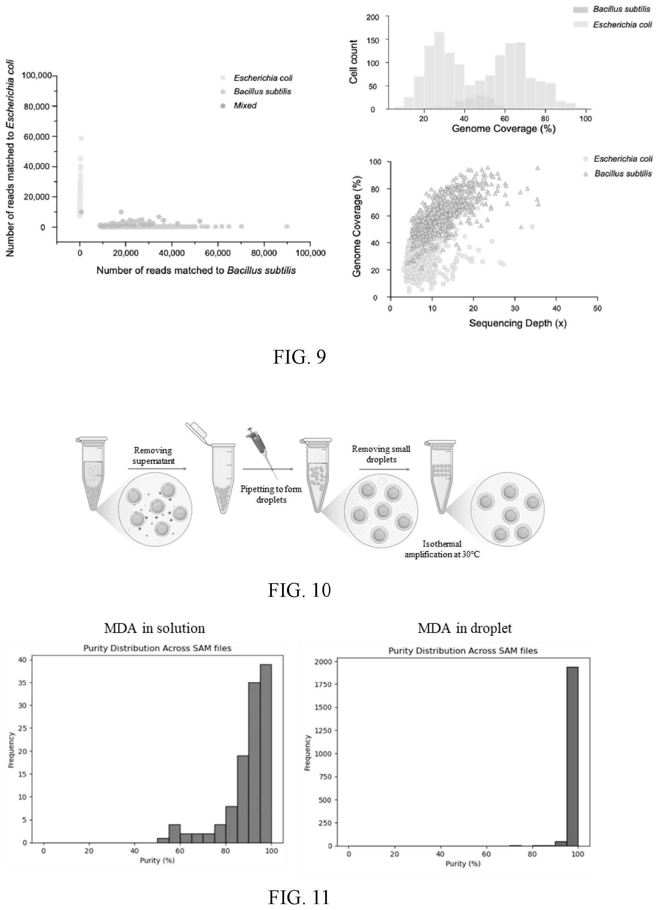

Whole-genome amplification was performed in the hydrogel microchambers:
Whole-genome amplification after droplet encapsulation of hydrogel microchambers: to prevent cross-contamination among different hydrogel microchambers during amplification, a pipetting method was used. As shown in Fig. 10, the hydrogel microchambers each encapsulating a microbial single-cell genome after lysis were mixed with whole-genome amplification reagents, and the mixture was pipetted repeatedly to form monodisperse droplets (monodisperse droplets could also be formed by vortexing or the like). Whole-genome amplification of the single microbial genome within each hydrogel microchamber was then performed in an isolated environment, which greatly improved sequencing purity and also simplified the operational steps. The specific procedure was as follows:
First, reagents for microbial single-cell whole-genome amplification in the hydrogel microchambers were prepared according to Table 3. 300 µL of the whole-genome amplification reagents was added to 300 µL of the hydrogel microchambers, mixed by pipetting, and allowed to stand for 1 min, such that the reagents for whole-genome amplification freely diffused from the exterior of the hydrogel microchambers into the interior thereof. Subsequently, the mixture was centrifuged for 1 min using a benchtop centrifuge. After excess upper liquid was removed, 600 µL of droplet-generation oil was added, and the mixture was pipetted for 2 min using a 200 µL pipette. After formation of monodisperse droplets was observed, the droplets were transferred to a constant-temperature apparatus at 30°C and reacted for 2 h. The amplification reaction was then terminated by incubation at 65°C for 10 min. Thereafter, 9.5 µL of the reaction product was mixed with 0.5 µL of SYBR Green solution, observed under a fluorescence microscope, and fluorescence images under excitation light at 488 nm were captured (Fig. 3c).

**Table 3 Whole-genome amplification reagent for hydrogel microchambers each encapsulating a microbial single cell**

| Component | Volume (µL) |
|---|---|
| 10 × Phi29 Reaction buffer | 60 |
| 10 U/µL Phi29 polymerase | 30 |
| 10 mM dNTP | 60 |
| Nuclease-Free Water | 36 |
| 500 µM Random primer(thermo, SO181) | 30 |
| 0.02U/µL Pyrophosphatase | 60 |
| 10v/v% Triton X-100 | 12 |
| 10v/v% Pluronic F68 | 12 |
| Total | 300 |

Fragmentation: The droplets were demulsified using PFO, and the permeable hydrogel microchambers containing microbial single-cell whole-genome amplification products were collected after being washed three times with DPBS buffer containing 0.1 v/v% Tween-20. A reaction system for fragmenting the whole-genome amplification products was then prepared according to Table 4. The reagents used for fragmentation were from the TruePrep^{®} DNA Library Prep Kit V2 for Illumina (Vazyme). 100 µL of the hydrogel microchambers containing microbial single-cell whole-genome amplification products was added to the fragmentation reagents. The Tn transposase (Vazyme TD501) bound to the DNA strands, randomly fragmented the whole-genome amplification products, and introduced the carried adapter sequences into the fragmented nucleic acid sequences of the substrate, thereby providing primer binding sites. The centrifuge tube was then placed on a thermostatic mixer and reacted at 55°C and 1500 rpm for 30 min. After completion of the reaction, the hydrogel microchambers containing the fragmented whole-genome amplification products were washed three times with a washing reagent and then stored at 4°C for subsequent single-cell indexing.

**Table 4 Reaction system for fragmenting the whole-genome amplification products in hydrogel microchambers**

| Component | Volume (µL) |
|---|---|
| 5 × TTBL | 40 |
| TTE V50 | 20 |
| Nuclease-Free Water | 40 |
| Total | 100 |

### Preparation of barcode beads:

A polyacrylamide hydrogel precursor solution was prepared as follows. 30 µL of H₂O, 100 µL of TBEST buffer, 150 µL of 40 w/v% acrylamide, 490 µL of 0.8 w/v% N,N'-bis(acryloyl)cystamine, 30 µL of 10 w/v% ammonium persulfate, and 200 µL of 250 µM acrydite-modified primer (acrydite-TTTCTACACGACGCTCTTCCGATCT, SEQ ID NO. 1) were added into a 1.5 mL centrifuge tube.

A microfluidic chip was used to introduce the hydrogel precursor solution into the corresponding channel. The droplets were collected and allowed to stand at room temperature for 6 h. After gelation, the droplets were demulsified to obtain hydrogel beads.

Subsequently, a split-pool method involving even random distribution and mixing was used to hybridize single oligonucleotide sequences onto the hydrogel beads. Through three rounds of split-pool, one oligonucleotide sequence segment was added to the hydrogel beads in each round of ligation reaction. After three rounds of reaction, a unique sequence combinatorially formed within each hydrogel bead was used as the barcode sequence. The barcode sequence was: TTTCTACACGACGCTCTTCCGATCTNNNNNNNACAGNNNNNNNGTCAN NNNNNNGTCTCGTGGGCTCGG (SEQ ID NO. 2), wherein NNNNNNN is a unique variable structure sequence of the barcode sequence. The three NNNNNNN segments are respectively barcode1, barcode2, and barcode3, wherein the sequence of barcode1 corresponds to BC1_A01-BC1_D01 in the table below, the sequence of barcode2 corresponds to BC2_A01-BC2_D01 in the table below, and the sequence of barcode3 corresponds to BC3_A01-BC3_D01 in the table below.

**Table 5 Variable structure sequence of the barcode sequence for hydrogel barcode beads**

| Name | Sequence | Name | Sequence | Name | Sequence |
|---|---|---|---|---|---|
| BC1_A01 | AAACCTG | BC2_A01 | AAACGTC | BC3_A01 | AAACAAC |
| BC1_A02 | AACCGCG | BC2_A02 | AAATCCA | BC3_A02 | AAAGATG |
| BC1_A03 | AACGTTG | BC2_A03 | AACCATG | BC3_A03 | AAATGAG |
| BC1_A04 | AAGACCT | BC2_A04 | AACTCAG | BC3_A04 | AACTCTT |
| BC1_A05 | AAGGTTC | BC2_A05 | AAGACCT | BC3_A05 | AAGGTTC |
| BC1_A06 | ACCAGTA | BC2_A06 | AATCGGT | BC3_A06 | AATCTTG |
| BC1_A07 | ACCGTAA | BC2_A07 | ACAACAC | BC3_A07 | ACACTGA |
| BC1_A08 | ACGATAC | BC2_A08 | ACACCGG | BC3_A08 | ACCGTAA |
| BC1_A09 | ACGCCGA | BC2_A09 | ACCAAGT | BC3_A09 | ACGATAC |
| BC1_A10 | ACTGATG | BC2_A10 | ACGAGGA | BC3_A10 | ACGCAGC |
| BC1_A11 | ACTTGTT | BC2_A11 | ACGCAGA | BC3_A11 | ACGGCCA |
| BC1_A12 | ACTTTCA | BC2_A12 | ACGGGTC | BC3_A12 | ACTACCT |
| BC1_B01 | AGAGCTT | BC2_B01 | ACGTAAA | BC3_B01 | ACTGAGT |
| BC1_B02 | AGCGTCG | BC2_B02 | ACTGAAC | BC3_B02 | AGAAAGG |
| BC1_B03 | AGCTCTC | BC2_B03 | ACTTTCA | BC3_B03 | AGAATCT |
| BC1_B04 | AGGCCAC | BC2_B04 | AGACATA | BC3_B04 | AGCGGTC |
| BC1_B05 | AGGGTGA | BC2_B05 | AGCCTAA | BC3_B05 | AGCTCCT |
| BC1_B06 | AGTAGTC | BC2_B06 | AGCGTCG | BC3_B06 | AGGAGGT |
| BC1_B07 | ATCACGA | BC2_B07 | AGCTATT | BC3_B07 | AGGGCTT |
| BC1_B08 | ATCTACT | BC2_B08 | AGGTGTA | BC3_B08 | AGTAGTC |
| BC1_B09 | ATGTGTG | BC2_B09 | AGTTACG | BC3_B09 | AGTGGGA |
| BC1_B10 | ATTCTAC | BC2_B10 | ATAAGAG | BC3_B10 | ATAAGGG |
| BC1_B11 | CAAGAAA | BC2_B11 | ATCATTC | BC3_B11 | ATCCGAA |
| BC1_B12 | CACACAA | BC2_B12 | ATGTGTG | BC3_B12 | ATTACTC |
| BC1_C01 | CACATTT | BC2_C01 | ATTGGAC | BC3_C01 | ATTCTAC |
| BC1_C02 | CAGAGAG | BC2_C02 | CAAGTTG | BC3_C02 | ATTTCTG |
| BC1_C03 | CAGCAGC | BC2_C03 | CACAAGG | BC3_C03 | CACAAAC |
| BC1_C04 | CAGCTAA | BC2_C04 | CACACCT | BC3_C04 | CACACTC |
| BC1_C05 | CAGTCCT | BC2_C05 | CACCTTA | BC3_C05 | CAGCAGC |
| BC1_C06 | CATTATC | BC2_C06 | CACTCCA | BC3_C06 | CAGTAAC |
| BC1_C07 | CCTACAC | BC2_C07 | CAGATCA | BC3_C07 | CATAACT |
| BC1_C08 | CGAACAT | BC2_C08 | CAGGTGC | BC3_C08 | CATATGG |
| BC1_C09 | CGAGAAG | BC2_C09 | CATATCG | BC3_C09 | CATTCGC |
| BC1_C10 | CGATGGC | BC2_C10 | CATTCGC | BC3_C10 | CGAACAT |
| BC1_C11 | CGCTTCA | BC2_C11 | CGAATGT | BC3_C11 | CGACTTC |
| BC1_C12 | CGGAGCT | BC2_C12 | CGACGTT | BC3_C12 | CGATTAT |
| BC1_D01 | CGTAGCG | BC2_D01 | CGATGTA | BC3_D01 | CGCGTTT |

The split-pool procedure was specifically as follows.

First, a first round of oligonucleotide sequence ligation was performed on the hydrogel beads. The collected hydrogel beads were evenly transferred into four 15 mL centrifuge tubes, with the volume of beads in each tube being about 5 mL. The hydrogel beads were washed with HBW. During each wash, the beads were centrifuged at 1,000 ×g for 3 min, and the supernatant was carefully removed. This washing step was repeated three times. The washed hydrogel beads were then combined into one new 15 mL centrifuge tube. The inner walls of the previously used 15 mL centrifuge tubes were rinsed with HBW to recover residual hydrogel beads, which were transferred into the new 15 mL centrifuge tube. The hydrogel beads in the new 15 mL centrifuge tube were centrifuged at 1,000 ×g for 1 min, and the supernatant was removed, thereby obtaining about 12 mL of hydrogel beads for subsequent experiments. Thereafter, 2.9 mL of 10× Isothermal Amplification Buffer was added into the centrifuge tube and mixed thoroughly by vortexing. The mixture was then centrifuged at 1,000 ×g for 1 min, and the supernatant was removed. Due to the effect of the high salt concentration, the hydrogel beads were dehydrated internally, leaving about 9.4 mL of hydrogel beads. Subsequently, a hydrogel bead premix was prepared according to Table 6 and transferred into a 25 mL reagent reservoir.

**Table 6 Hydrogel bead premix**

| Component | Volume (mL) |
|---|---|
| Nuclease-Free Water | 2.3 |
| Hydrogel bead | 9.4 |
| 10 mM dNTP | 1 |
| Total | 12.5 |

**Table 7 Program for hybridization reaction of hydrogel barcode beads**

| Temperature (°C) | Time (min) |
|---|---|
| 85 | 2 |
| 60 | 120 |
| 4 | Holding |

Subsequently, a multichannel pipette was used to transfer the hydrogel bead premix in the reagent reservoir into a round-bottom 96-well plate, with 35 µL added to each well. Then, a 96-well plate containing the first ligation primer was taken out. The first ligation primer was CTGTNNNNNNNAGAT. The 96-well plate was placed in a PCR instrument and reacted at 70°C (thermal lid: 105°C) for 40 s. The purpose of this step was to denature primer dimers. The 96-well plate containing the primer was then transferred to 4°C and held for 20 s. Afterwards, using a multichannel pipette, 30 µL of the hydrogel bead premix was transferred into a new 96-well PCR plate according to the numbering sequence on the plate, and 13.5 µL of the oligonucleotide (10 µM) from the 96-well plate containing the primer was added to each well. In the first ligation, the oligonucleotide was barcode1, and in the subsequent two repeated rounds, the oligonucleotides were barcode2 and barcode3, respectively. After thorough mixing, the 96-well PCR plate was placed in a PCR instrument, and a hybridization reaction was performed according to the program set forth in Table 7.

While the hybridization reaction was being performed, a premix required for isothermal amplification was prepared according to Table 8. During preparation, the centrifuge tube was inverted and mixed 15 times. After preparation, the premix was transferred into a 25 mL reagent reservoir and placed on ice. Using a multichannel pipette, the isothermal amplification premix was added to a new round-bottom 96-well plate, with 20 µL added to each well. The 96-well PCR plate after hybridization was removed from the PCR instrument, and 15 µL of the isothermal amplification premix was transferred into each well of the 96-well PCR plate using a multichannel pipette. After pipetting to mix thoroughly, the PCR plate was placed in a PCR instrument and reacted at 60°C for 1 h, thereby completing primer extension after hybridization. After completion of the hybridizationextension reaction, 40 µL of HBW buffer (10 mM Tris-HCl, pH 8.0, 400 µM EDTA, 0.1% Tween-20 (v/v)) was added to each well of the 96-well PCR plate to resuspend the hydrogel beads, and all liquid in the wells was transferred into a 15 mL centrifuge tube. The centrifuge tube was transferred to a centrifuge and centrifuged at 300 ×g for 15 min to collect the lower hydrogel beads. The hydrogel beads were washed with HBW buffer. During washing, HBW buffer was added into the 15 mL centrifuge tube, incubated with rotation at room temperature for 15 min, transferred to a centrifuge, and centrifuged at 300 ×g for 3 min. After the supernatant was removed, this step was repeated 3 times. The washed hydrogel beads could be stored briefly at 4°C.

**Table 8 Isothermal amplification premix during the preparation of hydrogel barcode beads**

| Component | Volume (mL) |
|---|---|
| Nuclease-Free Water | 6.85 |
| 10× isothermal amplification buffer | 0.8 |
| Bst 2.0 DNA polymerase (8,000 U/ml) | 0.35 |
| Total | 8 |

After completion, all hydrogel beads were mixed and washed to remove excess reagents. Subsequently, the double-stranded nucleic acid on the hydrogel beads needed to be denatured. Specifically, 5 mL of denaturation buffer (150 mM NaOH, 0.5% (w/w) Brij-35) was added to the hydrogel beads, reacted at room temperature for 10 min, and then centrifuged at 300 ×g for 3 min to remove the supernatant. Thereafter, the hydrogel beads were washed again with the denaturation buffer. After addition of the denaturation buffer to resuspend the hydrogel beads, the mixture was incubated at room temperature for 1 min, centrifuged at 300 ×g for 3 min, and the supernatant was discarded. This washing step was repeated 3 times. Afterwards, the hydrogel beads were washed twice with a neutralization buffer. Finally, the hydrogel beads were washed with TET buffer, centrifuged at 1,000 ×g for 3 min, and the supernatant was removed. This step was repeated 3 times, thereby removing excess oligonucleotide sequences and retaining only the oligonucleotide sequences ligated to the hydrogel beads. At this point, the first round of ligation was completed. The above procedure was repeated twice to complete preparation of the barcode beads, wherein the second ligation primer was TGACNNNNNNNCTGT, and the third ligation primer was CCGAGCCCACGAGACNNNNNNNTGAC (SEQ ID NO. 3).

After barcode sequences on the hydrogel beads had been completely synthesized, excess incomplete oligonucleotide strands needed to be removed by enzymatic digestion. The specific procedure was as follows. The prepared barcode beads were transferred into a 15 mL centrifuge tube, and an enzymatic digestion washing premix prepared according to Table 9 was added. The mixture was reacted at 37°C for 2 h. After completion of the reaction, the mixture was centrifuged at 300 ×g for 3 min to remove the supernatant, and the barcode beads were collected.

**Table 9 Enzymatic digestion washing premix**

| Component | Volume (mL) |
|---|---|
| Nuclease-Free Water | 14.7 |
| 10 ×FastDigest buffer | 1.5 |
| FastDigest HinfI | 0.3 |
| Total | 15 |

Next, the barcode beads were resuspended in a hybridization buffer and mixed thoroughly by vortexing. The mixture was then centrifuged at 1,000 ×g for 3 min, and the supernatant was carefully removed. This washing step was repeated 3 times. Thereafter, the barcode beads were transferred into a 50 mL centrifuge tube, and hybridization buffer was added to a volume of 22.5 mL. At the same time, 500 µL of an enzymatic digestion protection sequence (CCGAGCCCACGAGAC (SEQ ID NO. 6)) at a concentration of 1 mM was added into the centrifuge tube. The mixture was thoroughly mixed and placed on a rotating mixer to react at room temperature in the dark for 30 min. The purpose of this step was to allow the complete barcode sequences inside the hydrogel beads to form double strands with the added protection sequence, thereby preventing them from being digested by the subsequent nuclease Exo I.

Later, an endonuclease reaction system was prepared for the sufficiently hybridized hydrogel barcode beads according to Table 10. After preparation, the reaction system was placed on a rotating mixer at room temperature and reacted at room temperature in the dark for 2 h. After completion of the reaction, 17 mL of STOP-25 buffer (10 mM Tris-HCl (pH 8.0), 25 mM EDTA, 0.1% (v/v) Tween-20, 0.1 M KCl) was added to terminate the enzymatic digestion reaction. Subsequently, the added protection sequence for the beads was removed using a denaturation buffer. Finally, the filtered barcode beads were resuspended in Tris-EDTA buffer and stored at 4°C in the dark.

**Table 10 Endonuclease reaction system**

| Component | Volume (mL) |
|---|---|
| Hydrogel barcode bead | 11.5 |
| 10 × ExoI buffer | 3.3 |
| Nuclease-Free Water | 18 |
| ExoI (20 U/µL) | 0.44 |
| Total | 33 |

After synthesis of the hydrogel barcode beads had been completed, the integrity of the barcode sequences on the beads was verified using fluorescent probes. The designed probe sequences are shown in Table 11.

**Table 11 Probe sequences for examining hydrogel barcode beads**

| Namen | Sequence (5'-3') |
|---|---|
| Specific probe L1 (probe-a) | FAM-GGAAGAGCGTCGTGTAG (SEQ ID NO. 4) |
| Specific probe L2 (probe-b) | FAM-AGATCGGAAGAGCG (SEQ ID NO. 5) |
| Specific probe L3 (probe-c) | FAM-CCGAGCCCACGAGAC (SEQ ID NO. 6) |
| Non-specific probe (probe-n) | FAM-TCTGACGCTCAAATCAGTGG (SEQ ID NO. 7) |

The specific procedure was as follows. First, 1.4 mL of QC buffer was mixed with 40 µL of the hydrogel barcode beads, and the mixture was then centrifuged at 1,000 ×g for 1 min. The supernatant was carefully removed, and 120 µL of QC buffer was then added to resuspend the hydrogel beads. 36 µL aliquots of the hydrogel bead suspension were separately transferred into four new 1.5 mL centrifuge tubes. 4 µL of each of four probe solutions was added into the respective centrifuge tubes, wherein the concentration of each probe was 100 µM. The mixtures were vortexed thoroughly and reacted at room temperature in the dark for 20 min. After completion of the reaction, the hydrogel beads were washed 3 times with QC buffer. During each wash, the mixture was centrifuged at 1,000 ×g for 1 min, and the supernatant was carefully removed. After the final wash, the hydrogel beads were resuspended. Subsequently, 10 µL of the hydrogel bead suspension was transferred onto Countess^{™} Cell Counting Chamber Slides, and the fluorescence intensity distribution of the hydrogel beads was observed by confocal microscopy (Fig. 4a). The fluorescence intensity of the images was collected using ImageJ (Fig. 4b). The fluorescence differences after hybridization with the fluorescent probes (Fig. 4) demonstrate the sequence integrity of the barcode beads.

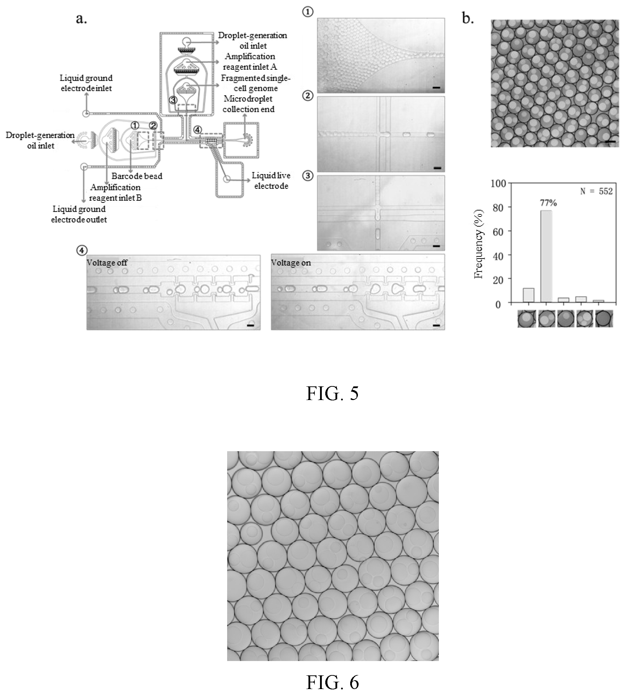

Single-cell indexing: In the red structural region of the chip shown in Fig. 5a, polyacrylamide beads carrying barcode sequences were encapsulated together with an aqueous phase in droplets. In the black structural region of the chip, permeable hydrogel microchambers containing fragmented single-cell genomic DNA and a PCR solution (containing DTT) were encapsulated in droplets. The two types of droplets met and were paired in a 1:1 manner, with a pairing efficiency of up to 77% (Fig. 5b), and were fused upon application of an electric field. As a result, one barcode bead and one permeable hydrogel microchamber were co-encapsulated in a single droplet (Fig. 6). In Fig. 5, images ①-④ show the hydrogel encapsulation and droplet fusion process captured using a high-speed camera. The droplets were then subjected to amplification in a PCR instrument.

**Table 12 PCR solution for linear amplification**

| Component | Volume (µL) |
|---|---|
| Phusion High-Fidelity Reaction Buffer (M0530L, NEB) | 120 |
| Phusion High-Fidelity DNA Polymerase (M0530L, NEB) | 16 |
| Deep Vent DNA Polymerase (M0258L, NEB) | 16 |
| ET-SSB (M2401S, NEB) | 12 |
| 100 mM DTT | 64 |
| 10 × PCR stabilizer | 64 |
| 10 mM dNTP | 64 |
| Nuclease-Free Water | 16 |
| Total | 348 |

**Table 13 PCR program for linear amplification of droplets**

| Temperature (°C) | Time | Cycle |
|---|---|---|
| 72 | 5 min | 1 |
| 98 | 3 min | 1 |
| 98 | 10 s | 15 |
| 60 | 30 s | |
| 72 | 5 min | |
| 4 | Holding | - |

Because the droplets contained PCR reaction reagents and DTT capable of dissolving the barcode beads, after dissolution of the barcode beads, the barcode sequences were released from the beads and entered the permeable hydrogel microchambers. Since the droplets contained only primers of barcode, the genomes in the permeable hydrogel microchambers were labeled with barcodes by linear amplification. Since each barcode bead carried a different barcode sequence, it could be determined from the barcode sequence whether genomes originated from the same cell, thereby achieving single-cell indexing.

After completion of the reaction, the droplets were demulsified and the permeable hydrogel microchambers were collected. The permeable hydrogel microchambers were washed 3-5 times with DPBS buffer containing 0.1 v/v% Tween-20 to remove excess oligonucleotide strands and enzymes or metal ions introduced during the reaction process.

Finally, the permeable hydrogel microchambers were combined into a 1.5 mL centrifuge tube and dissolved with 1 M NaOH of twice the volume of hydrogel microchambers. The pH was then neutralized to neutrality with 1 M acetic acid, thereby dissolving the hydrogel microchambers. At this stage, the DNA was present in the aqueous solution. 0.5× VAHTS Clean Beads was added to purify the linear amplification products, followed by enrichment by exponential amplification (i.e., by adding forward and reverse primers).

The reaction system for the enrichment amplification was prepared according to Table 14. The sequence primer R was:
CTACACGACGCTCTTCCGATCT (SEQ ID NO. 8), and the sequence of primer F was: TGGTCGGCAGCGTC (SEQ ID NO. 9).

**Table 14 PCR solution for the enrichment amplification**

| Component | Volume (µL) |
|---|---|
| KAPA HiFi Hotstart MasterMix (Roche, KK2600) | 25 |
| 10µM Primer F | 5 |
| 10µM 2^{nd} PCR primer R | 5 |
| Linear amplification product | 10 |
| Nuclease-Free Water | 5 |
| Total | 50 |

**Table 15 PCR program for the enrichment amplification**

| Temperature (°C) | Time | Cycle |
|---|---|---|
| 98 | 3 min | 1 |
| 98 | 10 s | 15 |
| 60 | 30 s | |
| 72 | 5 min | |
| 72 | 5 min | 1 |
| 4 | Holding | - |

A Bioanalyzer was used for quality control of the library fragment size distribution before sequencing.

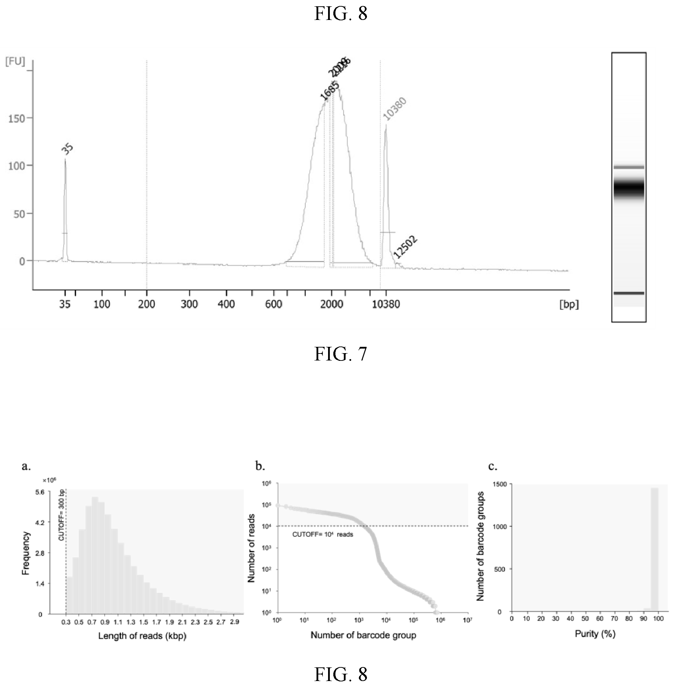

Using Example 1 as a representative example, Fig. 7 shows the fragment length distribution of the prepared DNA library.

Using the Nanopore ligation kit (SQK-LSK114), adapters required for third-generation sequencing were added to the indexed genomic DNA according to the experimental procedure provided by the supplier. After completion of the reaction, third-generation sequencing was performed using the Nanopore MinION platform. The results are shown in Figs. 8 and 9.

The results shown in Fig. 8 indicate that, for more than 95% of the single cells, the single-cell purity is higher than 95%. Fig. 9 shows that the single-cell genome coverage can reach higher than 80%. At present, microbial single-cell whole-genome sequencing technologies mainly include SiC-seq and Microbe-seq, wherein SiC-seq achieves about 1% single-cell genome coverage, and Microbe-seq achieves about 5%-10% single-cell genome coverage.

### Example 4

In this example, the methods for single-cell encapsulation, lysis, and whole-genome amplification were the same as those in Example 1. The main difference was that, in this example, the barcode sequence was ligated to the fragmented genome by an enzymatic ligation method to achieve indexing.

For fragmentation, the Tn5 transposome complex used herein was prepared in-house. Specifically, a commercial Tn5 transposase was purchased, phosphorylated primers were synthesized, and then loaded onto the Tn5 transposase. After preparation of the Tn5 transposase carrying the phosphorylated adapters, the whole-genome amplification products were fragmented using the same method as in Example 1.

The specific procedure for preparing the Tn5 transposome complex was as follows:

### 1. Preparation of Adapter Mix

Primer sequences: the underlined sequence portions of Primer B' and Primer C' were reverse complementary to Primer A.
Primer A: 5'-Phos-CTGTCTCTTATACACATCT-NH2-3' (SEQ ID NO. 10);
Primer B': 5'-TCGTCGGCAGCGTCAGATGTGTATAAGAGACAG-3' (SEQ ID NO. 11);
Primer C': 5'-phos-GTCGTCGTGGGCTCGGAGATGTGTATAAGAGACAG-3' (SEQ ID NO. 12);

### Preparation of Adapter Mix:

### Reactant 1:

Adapter1: Primer A (10 µM) 10 µL, Primer B' (10 µM) 10 µL;

### Reactant 2:

Adapter2: Primer A (10 µM) 10 µL, Primer C (10 µM) 10 µL;
Reactants 1 and 2 were mixed at equal volumes and subjected to the following program to obtain Adapter Mix:

**Table 16**

| Thermal lid | on, 105°C |
|---|---|
| 75°C | 15min |
| 60°C | 10min |
| 50°C | 10min |
| 40°C | 10min |
| 25°C | 35min |

### 2. Loading of Adapter Mix

A reaction system was prepared according to Table 17, followed by reaction at 30°C for 1 h. The reaction product was designated TTE Mix.

**Table 17**

| Component | 2µg preparation system |
|---|---|
| TruePrep Tagment Enzyme (500 ng/µl) | 4 µl |
| Adapter Mix | 7 µl |
| Coupling Buffer | 39 µl |
| Total | 50 µl |

DNA fragmentation: a fragmentation reaction system was prepared according to Table 18, followed by reaction at 55°C for 30 min to fragment the whole-genome DNA.

**Table 18 Fragmentation reaction system**

| Component | Volume (µL) |
|---|---|
| 5 ×Tagment buffer L | 10 |
| TTE Mix | 10 |
| dd H₂O | 30 |
| Total | 50 |
| gel | 25 µl |

During preparation of the barcode beads, it was only necessary to introduce a phosphorylated primer in the final round of barcode synthesis. All other operating methods were the same as those in Example 1.

### The ligation-based indexing reaction system is shown in the table below.

**Table 19 Ligation-based indexing reaction system**

| Component | Volume (µL) |
|---|---|
| StickTogether DNA ligase reaction buffer | 164 |
| T7 DNA ligase | 16 |
| 100 mM DTT | 64 |
| 10 × PCR stabilizer | 64 |
| 10 mM dNTP | 64 |
| Total | 372 |

### Reaction program: 25°C, 16 h

After completion of the ligation reaction, enrichment amplification (exponential amplification) was performed. The reaction system and reaction program are shown in the table below, wherein the primer sequences were the same as those in Example 1.

**Table 20 PCR solution for the enrichment amplification**

| Component | Volume (µL) |
|---|---|
| 5 × TAB | 10 |
| 10µM Primer F | 5 |
| 10µM 2^{nd} PCR primer R | 5 |
| TAE | 1 |
| Nuclease free water | 4 |
| Hydrogel microchambers | 25 |
| Total | 50 |

**Table 21 PCR program for the enrichment amplification**

| Temperature (°C) | Time | Cycle |
|---|---|---|
| 72 | 5 min | 1 |
| 98 | 3 min | 1 |
| 98 | 15s | 15 |
| 60 | 30 s | |
| 72 | 3 min | |
| 72 | 5 min | 1 |
| 4 | Holding | - |

After completion of the amplification, the hydrogel microchambers were dissolved, the amplification products were purified, and sequencing was performed (the specific method was the same as that in the examples above).

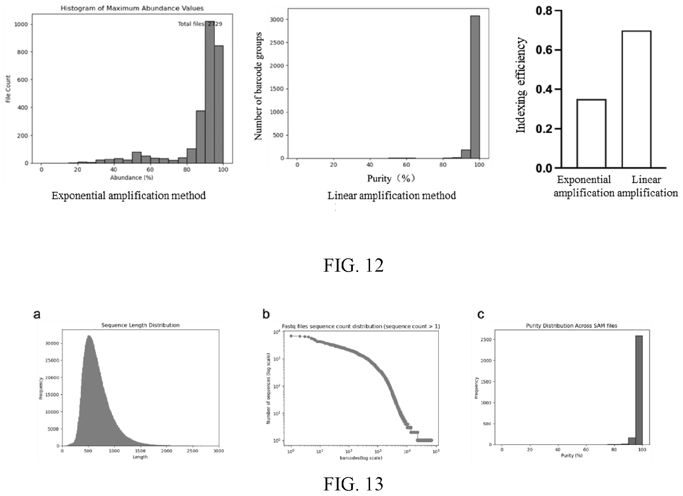

The results of ligation-based indexing are shown in Fig. 13, which shows that a relatively high single-cell purity was obtained.

### Comparative Example 1

Lysis Method 1: A commercially purchased prepGEM Bacteria kit (MicroGEM) was used. A lysis reaction system was prepared according to Table 22 and mixed with the hydrogel microchambers. The mixture was incubated sequentially in a shaking mixer at a shaking speed of 1500 rpm at 37°C for 30 min, 75°C for 10 min, and 95°C for 5 min. After the reaction solution had cooled to room temperature, the mixture was centrifuged at 5000 rpm for 1 min to remove the supernatant. The hydrogel microchambers were then washed five times with a Tris-EDTA solution containing 0.1% Tween-20 (v/v) to remove lysis buffers, cell debris, and other impurities.

**Table 22 Lysis buffer for microbial single cells in hydrogel microchambers**

| Component | Volume (µL) |
|---|---|
| Nuclease-Free Water | 280 |
| 10 × Green Buffer (MicroGEM, PBA0100) | 100 |
| 300U/ul Lysozyme | 10 |
| prepGEM (MicroGEM, PBA0100) | 10 |
| 10 × Enhancer (MicroGEM, PBA0100) | 100 |
| Total | 500 |
| Hydrogel microchambers (each containing a microbial single cell) | 500 |

Lysis Method 2: A first-step reaction system was prepared according to Table 23. The solution and the microchambers were reacted overnight in a thermostatic mixer at 37°C and 1500 rpm. The mixture was then centrifuged at 5000 rpm for 1 min to remove the supernatant, and washed twice with a DPBS solution containing 0.1% Tween-20 (v/v). Next, a second-step lysis was performed according to the reaction system shown in Table 24, and the mixture was reacted in a thermostatic mixer at 55°C and 1500 rpm for 30 min. After the reaction solution had cooled to room temperature, the mixture was centrifuged at 5000 rpm for 1 min to remove the supernatant, and then washed twice with an aqueous solution containing 2% Tween-20 (v/v) to quench SDS in the solution. Next, the hydrogel microchambers were washed once with absolute ethanol to inactivate proteinase K and avoid affecting downstream amplification reactions. Finally, the hydrogel microchambers were washed five times with a Tris-EDTA solution containing 0.1% Tween-20 (v/v), stored in that solution, and kept temporarily in a refrigerator at 4°C for subsequent whole-genome amplification.

**Table 23 Lysis buffer A for microbial single cells in hydrogel microchambers**

| Component | Volume (µL) |
|---|---|
| Nuclease-Free Water | 365 |
| 1M NaCl | 10 |
| 50 mM EDTA | 50 |
| 1M DTT | 10 |
| 500 U/mL Lysostaphin | 20 |
| 2500 U/mL Mutanolysin | 40 |
| 300 U/µL Lysozyme Solution | 3 |
| 2U/µL Zymolyase | 2 |
| Total | 500 |
| Hydrogel microchambers (each containing a microbial single cell) | 500 |

**Table 24 Lysis buffer B for microbial single cells in hydrogel microchambers**

| Component | Volume (µL) |
|---|---|
| Nuclease-Free Water | 40 |
| 1M NaCl | 90 |
| 50 mM EDTA | 180 |
| 100 mM Tris-HCl (pH 8.0) | 90 |
| 5w/v% SDS | 90 |
| 20 mg/mL proteinase K | 10 |
| Total | 500 |
| Hydrogel microchambers (each containing a microbial single cell) | 500 |

The results are shown in the table below.

**Table 25**

| Lysis method | *Escherichia coli*: *Bacillus subtilis* |
|---|---|
| Comparative examplelysis method 1 | 12:1 |
| Comparative examplelysis method 2 | 10:1 |
| Example 1 | 1:1-2:1 |

The results show that, compared with the lysis scheme in Example 1, Lysis Methods 1 and 2 in this comparative example lead to insufficient lysis and exhibit lysis bias, thereby causing a large difference in the proportions of *Escherichia coli* and *Bacillus subtilis* obtained by sequencing, which is unfavorable for mixedbacteria detection.

### Comparative Example 2

This comparative example differed from Example 1 only in that the linear amplification used in the DNA indexing was replaced with exponential amplification. Specifically, an additional primer for amplification from the other end (Primer F: TGGTCGGCAGCGTC (SEQ ID NO. 9)) was added to the linear amplification reaction system of Example 1, thus indexing was achieved by exponential amplification, while all other conditions remained unchanged.

**Table 26 Exponential amplification reaction system**

| Component | Volume (µL) |
|---|---|
| Phusion High-Fidelity Reaction Buffer | 120 |
| Phusion High-Fidelity DNA Polymerase | 16 |
| Deep Vent DNA Polymerase | 16 |
| Primer F (100 µM) | 4 |
| 100 mM DTT | 64 |
| 10 × PCR stabilizer | 64 |
| 10 mM dNTP | 64 |
| Nuclease-Free Water | 24 |
| Total | 372 |

The results show that, compared with linear amplification, the exponential amplification method affects single-cell purity and has a lower indexing efficiency (Fig. 12).

### Comparative Example 3

In this comparative example, the hydrogel beads (also referred to as hydrogel microchambers) after lysis were not formed into microdroplets. Instead, multiple displacement amplification (MDA) was used to directly achieve single-cell whole-genome amplification (WGA) of the permeable hydrogel beads in an aqueous solution system.

First, a solution for performing microbial single-cell whole-genome amplification in the hydrogel microchambers was prepared according to Table 27. The solution was mixed thoroughly by pipetting, then placed in a thermostatic shaking mixer and reacted at 1500 rpm and 30°C for 2 h. Thereafter, the mixture was incubated in a metal bath at 65°C for 10 min to inactivate phi29 DNA polymerase, thereby terminating the amplification reaction.

**Table 27 Reaction system for microbial single-cell whole-genome amplification in hydrogel microchambers**

| Component | Volume (µL) |
|---|---|
| 10 × Phi29 Reaction buffer | 60 |
| 10 U/µL Phi29 polymerase | 30 |
| dNTP mix (10 mM each) | 60 |
| Random primer (500 µM) (thermo, SO181) | 30 |
| Pyrophosphatase (0.02 U/µL) | 60 |
| Nuclease-Free Water | 60 |
| Hydrogel microchambers containing microbial single-cell lysis products | 300 |
| Total | 600 |

The results are shown in Fig. 11. The results show that, compared with whole-genome amplification in an aqueous solution in Comparative Example 3, the single-cell purity obtained by whole-genome amplification using encapsulated hydrogel droplets in Example 1 is significantly higher.

In summary, the method of the present disclosure was used to perform microbial single-cell whole-genome amplification, indexing, and library construction on an artificial mixed bacterial sample prepared by mixing *Escherichia coli* and *Bacillus subtilis* at an equal ratio. The results show that the method can lyse Gram-negative bacteria and Gram-positive bacteria without bias, achieves a single-cell purity of greater than 95% for more than 95% of single cells, and provides a single-cell genome coverage of up to 80%.

The above embodiments are merely illustrative of the principles and effects of the present disclosure, and are not intended to limit the present disclosure. Any person skilled in the art may make modifications or changes to the above embodiments without departing from the spirit and scope of the present disclosure. Therefore, all equivalent modifications or changes made by those of ordinary skill in the art without departing from the spirit and technical concept disclosed herein shall fall within the scope of the claims of the present disclosure.

## Claims

1. A method for constructing a microbial single-cell whole-genome sequencing library, comprising the following steps:
1) providing a microchamber containing a microbial single cell, lysing the single cell, and amplifying a whole genome of the microbial single cell;
2) fragmenting the whole genome obtained in step 1), thereby obtaining a microchamber containing a fragmented genome;
3) introducing a barcode bead into the microchamber obtained in step 2), and barcoding the fragmented genome, thereby obtaining a microchamber containing a barcoded whole genome; and
4) dissolving the microchamber obtained in step 3), and performing enrichment amplification of the barcoded whole genome, thereby obtaining the microbial single-cell whole-genome sequencing library.

2. The construction method according to claim 1, wherein a method for lysing the single cell comprises one or more of an enzymatic lysis method, an alkaline lysis method, and a heat-shock method;
preferably, an enzyme used in the enzymatic lysis method is one or more of Labiase lysing enzyme, lysostaphin, lysozyme, mutanolysin, zymolyase, and proteinase K;
preferably, reagents for lysing the single cell comprise a lysis buffer A and a lysis buffer B;
preferably, the lysis buffer A comprises components at the following working concentrations: 30-50 U/mL lysostaphin, 80-120 U/mL mutanolysin, 0.5-4 U/µL lysozyme, 0.005-0.02 U/µL zymolyase, 30-70 mM NaCl, 3-8 mM EDTA, 5-15 mM Tris-HCl, and 0.1-1 v/v% Triton X-100;
preferably, the lysis buffer B comprises components at the following working concentrations: 0.1-0.5 mg/mL proteinase K, 80-120 mM NaCl, 5-15 mM EDTA, 5-15 mM Tris-HCl, and 0.01-2 w/v% SDS;
preferably, steps of lysing the single cell comprise mixing the lysis buffer A with the microchamber to perform a first lysis, washing, and then adding the lysis buffer B to perform a second lysis;
preferably, conditions for the first lysis are 30-43°C, 1300-1800 rpm, and/or 8-16 h;
preferably, conditions for the second lysis are 50-65°C, 1300-1800 rpm, and/or 0.5-2 h.

3. The construction method according to claim 1, wherein the microchamber comprises a gel bead or a gel droplet;
preferably, the microchamber comprises an inner layer and an outer layer, the inner layer comprises a hydrogel of a first polymer, and the outer layer comprises a hydrogel of a second polymer;
preferably, the first polymer and the second polymer are each independently selected from dextran, high-molecular-weight poly(ethylene glycol) diacrylate, polyacrylamide, agarose, gelatin methacryloyl, four-arm polyethylene glycol, eightarm polyethylene glycol, polyethylene glycol-maleimide, and thiol-polyethylene glycol-thiol.

4. The construction method according to claim 1, wherein the barcode bead comprises at least one of a polyacrylamide bead, a polystyrene bead, a poly(ethylene glycol) diacrylate bead, a poly(lactic-co-glycolic acid) bead, an agarose bead, and a magnetic bead;
preferably, when the microchamber in amplifying the whole genome of the microbial single cell is a gel droplet, the gel droplet is formed by a microfluidic chip, a pipetting method, or a vortexing method; preferably, the gel droplet is formed by the pipetting method or the vortexing method;
preferably, amplification of the whole genome of the microbial single cell and the enrichment amplification are exponential amplification or linear amplification.

5. The construction method according to claim 1, wherein barcoding the fragmented genome in step 3) comprises barcoding the fragmented genome by a DNA ligase or by single-primer amplification;
preferably, the single-primer amplification is linear amplification;
preferably, before barcoding the fragmented genome, the construction method further comprises releasing a barcode from the barcode bead;
preferably, a method for releasing the barcode from the barcode bead comprises chemical bond cleavage, enzymatic degradation, or ultraviolet cleavage;
preferably, the chemical bond comprises a disulfide bond;
preferably, a reagent for the chemical bond cleavage is one or more of DTT, dichloromethane, ethyl acetate, acetone, PBS, and a degradation enzyme.

6. A microbial single-cell whole-genome sequencing library, which is obtained by the construction method according to any one of claims 1-5.

7. Use of the method for constructing the microbial single-cell whole-genome sequencing library according to any one of claims 1-5, or of the microbial single-cell whole-genome sequencing library according to claim 6, in microbial single-cell sequencing.

8. A method for microbial single-cell whole-genome sequencing, comprising:
a) constructing a sequencing library according to the construction method according to any one of claims 1-5; and
b) sequencing the sequencing library provided in step a);
preferably, the sequencing comprises next-generation sequencing or thirdgeneration sequencing.

9. A product for constructing a microbial single-cell whole-genome sequencing library, comprising the barcode bead, a reagent for lysing the single cell, a reagent for amplifying the whole genome of the microbial single cell, a fragmentation reagent, a reagent for barcoding the fragmented genome, and a reagent for enrichment amplification in the construction method according to any one of claims 1-5;
preferably, the product is a kit, a chip, or a detection system.

10. Use of the method for constructing the microbial single-cell whole-genome sequencing library according to any one of claims 1-5, or of the microbial single-cell whole-genome sequencing library according to claim 6, or of the method for microbial single-cell whole-genome sequencing according to claim 8, or of the product according to claim 9, in preparing a detection kit, a detection device, or a detection system for microbial research.
